(19)

Europäisches
Patentamt
European
Patent Office
Office européen
des brevets

(11)  **EP 3 777 653 A1**

(12)  **EUROPEAN PATENT APPLICATION**
published in accordance with Art. 153(4) EPC

(43) Date of publication:
**17.02.2021 Bulletin 2021/07**

(21) Application number: **18911781.5**

(22) Date of filing: **06.04.2018**

(51) Int Cl.:
*A61B 5/00* (2006.01)   *A61B 5/145* (2006.01)
*G06N 3/08* (2006.01)

(86) International application number:
**PCT/KR2018/004088**

(87) International publication number:
**WO 2019/189977 (03.10.2019 Gazette 2019/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.03.2018 KR 20180036160**

(71) Applicant: **i-Sens, Inc.**
**Seoul 06646 (KR)**

(72) Inventors:
• **KANG, Byeong Keun**
**Seoul 06113 (KR)**
• **LEE, Myeongho**
**Seoul 04103 (KR)**
• **LEE, Seok-Won**
**Seongnam-si, Gyeonggi-do 13554 (KR)**
• **NAM, Hakhyun**
**Seoul 01227 (KR)**
• **CHA, Seonghun**
**Seoul 08809 (KR)**

(74) Representative: **Arnold & Siedsma**
**Bezuidenhoutseweg 57**
**2594 AC The Hague (NL)**

(54)  **ARTIFICIAL NEUTRAL NETWORK DEEP LEARNING-BASED METHOD, APPARATUS, LEARNING STRATEGY, AND SYSTEM FOR ANALYTE ANALYSIS**

(57)    The present invention relates to an artificial neural network deep learning-based analyte analysis system utilizing a system, the system including a biometric information measurer, and a biometric information analysis artificial intelligence-based deep learning server that includes: a signal acquiring portion acquiring a signal through an electrochemical reaction, which occurs due to injection of blood collected by the biometric information measurer into a strip that includes a pair of electrodes; a signal processor pre-processing the signal acquired by the signal acquiring portion as a signal for artificial intelligence deep learning; a biometric information measurement algorithm generator that automatically extracts features by using an artificial neural network deep learning-based method for a biometric information measurement algorithm optimized using the signal having been processed through the signal processor; and an optimized algorithm result providing portion that provides the optimized biometric information measurement algorithm to the biometric information measurement apparatus.

FIG. 1

**Description**

**Technical Field**

The present invention relates to an analyte analysis method, an apparatus, a learning method, and a system utilizing an artificial neural network deep learning-based method. More particularly, the present invention relates to an analyte analysis method, an apparatus, a learning method, and a system utilizing an artificial neural network deep learning-based method that recognizes an area corresponding to an analyte from data imaged by converting a signal acquired from a sensor by applying an artificial neural network deep learning strategy, and predicting the most appropriate type and concentration of analyte by extracting elements required for determination of an analyte type and concentration in the corresponding area.

**Background Art**

In general, one of the most important criteria for in-vitro diagnosis products is the accuracy of the measurement results.

In such products, when an analyte is present, a detection sensor outputs a signal, and an analyzer recognizes it and applies a predetermined calibration curve or algorithm to send quantitative or qualitative results. The accuracy of the measurement result can be influenced by the interference action caused by various variables such as an interferent, an external environment, and sample properties.

In case of an electrochemical sensor utilized for blood glucose measurement, an output electrical signal may be influenced due to a change in a diffusion coefficient toward an electrode of blood or a reaction rate at an electrode surface due to a change in blood properties such as presence of a material other than an analyte oxidized at the electrode surface or viscosity. In order to minimize or eliminate such effects, instead of a method applying a simple DC voltage to the sensor, a method that applies an input voltage of a waveform having various types of patterns, and processing the waveform obtained from the electrode reaction with appropriate statistical mathematical techniques, has been used.

Since the 2010s, the blood glucose measurement market has increased in the number of companies entering this business, and the pressure to cut prices has increased due to the expansion of national insurance in each country, while major standards management organizations and authentication organizations are strengthening the standards for accuracy and precision day by day to increase user safety. For example, the amount of insurance paid by Medicare insurance in the United States has fallen to less than half due to competitive bidding that began since 2008. However, the FDA New Guidance, recently published in 2016, contains much stronger requirements for accuracy and the limitation of interference effects of various interference materials than the specifications of existing blood glucose measurement apparatus (https://www.fda.gov/downloads/medicaldevices/deviceregulationandguidance/g uidancedocuments/ucm380325.pdf).

As described, competition is intensified, and the demand for performance of the measurement system causes a need develop a system with low cost and high performance in order to survive in the market for enhanced blood glucose measurement, and this contradictory development goal can only be achieved through excellent technological progress.

To lower the production cost of the blood glucose measurement system, limited performance hardware (e.g., STM8L Series MCU) and simple structured sensor strip are required. On the other hand, data can be transmitted and operated via Bluetooth to enable the function of a high-performance chip or smart phone to use a method that requires a high-level operation such as deep learning to improve the performance of the blood glucose measurement system, and a system that communicates with a system or a cloud system to perform separate calculations is required, but this goes against the current market trend. Although an algorithm that requires a large amount of operation, such as a deep learning algorithm, is implemented on limited hardware, there is still a problem that takes a long time for blood glucose measurement. To be competitive in the current blood glucose measurement device market, it is necessary to be able to output estimated blood glucose concentration within about 8 seconds after the analyte input. In addition, it is advantageous to continue using the strip with only a pair of electrodes made in the existing production process to minimize the production cost of the blood glucose strip. Meanwhile, it is difficult to accurately estimate glucose in the blood using only information obtained from the electrode with the limited structure and the existing signal processing method.

**Disclosure**

**Technical Problem**

The present invention is proposed to solve such a problem, and provides an analyte analysis method, an apparatus, and a system utilizing an artificial neural network deep learning-based method that can improve the accuracy, precision, and interference correction performance including hematocrit of an algorithm, unlike an existing multiple linear regression method, and is based on an optimized blood glucose estimation and an artificial intelligence-based deep learning technology that can be implemented with meter hardware with a limited structure and a sensor strip.

In addition, the purpose of the present invention is to provide an analyte analysis method, an apparatus, and a system utilizing an artificial neural network deep learning-based method with data dimension reduction and an algorithm structure reduction to thereby implement complex algorithms even on limited hardware.

In addition, the purpose of the present invention is to provide an analyte analysis method, an apparatus, and a system utilizing an artificial neural network deep learning-based method that can efficiently perform calculations and minimize operation time within 8 seconds suitable for blood glucose measurement even if an algorithm with a large amount of operations is used.

In addition, the purpose of the present invention is provide an analyte analysis method, an apparatus, and a low-cost system utilizing an artificial neural network deep learning-based method that can obtain the effect of using multiple electrodes through a technology that can extract additional information inherent in data to the maximum from information obtained through a limited number of electrodes.

## Technical Solution

The present invention relates to n analyte analysis system utilizing an artificial neural network deep learning-based method, including: a biometric information measurer, and a biometric information analysis artificial intelligence-based deep learning server that includes: a signal acquiring portion acquiring a signal through an electrochemical reaction, which occurs due to injection of blood collected by the biometric information measurer into a strip that includes a pair of electrodes; a signal processor pre-processing the signal acquired by the signal acquiring portion as a signal for artificial intelligence deep learning; a biometric information measurement algorithm generator that automatically extracts features by using an artificial neural network deep learning-based method for a biometric information measurement algorithm optimized using the signal having been processed through the signal processor; and an optimized algorithm result providing portion that provides the optimized biometric information measurement algorithm to the biometric information measurement apparatus.

A blood glucose measurement apparatus utilizing an artificial neural network deep learning-based method according to another aspect of the present invention includes: a connector equipped with an electrochemical biosensor having a pair of electrodes; a current-voltage converter that is electrically connected with the connector; a digital-analog converter circuit that is controlled to apply a constant voltage to the pair of electrodes of the electrochemical biosensor, and a cyclic voltage having a triangular waveform, a square waveform, a staircase waveform, or other modified trigonometric function waveform; and a microcontroller that controls the connector and the digital-analog converter, wherein the microcontroller includes an artificial intelligence deep learning algorithm calculator that automatically calculate blood glucose according to an optimized artificial intelligence-based deep learning algorithm acquired from the analyte analysis system utilizing an artificial neural network deep learning-based method.

A blood glucose measurement method utilizing an artificial neural network deep learning-based method according to an exemplary embodiment of the present invention includes: preparing a sample; loading an electrochemical biosensor into a biometric information measurer; applying a constant voltage when the biosensor contacts the sample and thus blood wets an operation electrode and an auxiliary electrode of the electrochemical biosensor, and continuously applying a cyclic voltage having a triangular waveform, a square waveform, a staircase waveform, or other modified trigonometric function waveform at a chronical voltage application termination point after the application of the constant voltage; and calculating a blood glucose value measured value from a response current measured by an optimized artificial intelligence deep learning blood glucose measurement algorithm.

A biometric information analysis artificial intelligence-based deep learning method according to another aspect of the present invention includes: acquiring a signal through an electrochemical reaction that occurs due to injection of blood collected through a biometric information measurer to a strip having a pair of electrodes; processing the acquired signal as a signal for artificial intelligence deep learning; forming an algorithm structure formed of a signal feature extraction portion and a blood glucose prediction portion for blood glucose measurement; learning a blood glucose measurement algorithm by adjusting all variables in the algorithm to minimize a difference between a blood glucose prediction value and a true value; and performing optimization.

## Advantageous Effects

An analyte analysis method, an apparatus, and a system utilizing an artificial neural network deep learning-based method according to an exemplary embodiment of the present invention can improve the accuracy, precision, and interference correction performance including hematocrit of an algorithm, unlike an existing multiple linear regression method, and can provide an optimized blood glucose estimation and an artificial intelligence-based deep learning strategy that can be implemented with meter hardware with a limited structure and a sensor strip.

In addition, according to the analyte analysis method, the apparatus and the system utilizing the artificial neural network deep learning-based method of the exemplary embodiment of the present invention, in an exemplary embodiment of the present invention, data dimension reduction and algorithm structure reduction can be carried out to thereby implement complex algorithms even on limited hardware.

In addition, according to the analyte analysis method, the apparatus, and the system utilizing the artificial neural network deep learning-based method of the exemplary embodiment of the present invention, calculations can be effectively performed and thus operation time can be reduced within 8 seconds suitable for blood glucose measurement even if an algorithm with a large amount of operations is used.

The analyte analysis method, the apparatus, and the system utilizing the artificial neural network deep learning-based method, according to the exemplary embodiment of the present invention, can obtain the effect of using multiple electrodes through a technology that can extract additional information inherent in data to the maximum from information obtained through a limited number of electrodes.

**Description of the Drawings**

FIG. 1 is a schematic view of an analyte analysis system utilizing an artificial neural network deep learning method according to an exemplary embodiment of the present invention.

FIG. 2 is a schematic diagram of the biometric information analysis artificial intelligence-based deep learning server in an analyte analysis learning system that utilizes the artificial neural network deep learning-based method according to the exemplary embodiment of the present invention.

FIG. 3 is an internal schematic diagram of a blood glucose measurement apparatus utilizing the artificial neural network deep learning-based method according to the exemplary embodiment of the present invention.

FIG. 4 is a flowchart of a blood glucose measurement method utilizing the artificial neural network deep learning-based method according to the exemplary embodiment of the present invention.

FIG. 5 is a graph that shows clinic test accuracy performance according to the existing method and the proposed method.

FIG. 6 is a graph that shows noise sensitivity evaluation according to the existing method and the proposed method.

FIG. 7 shows a voltage and a response current applied in a biometric information analysis artificial intelligence-based deep learning method according to an exemplary embodiment of the present invention, FIG. 8 is an exemplary view of an abnormal signal, FIG. 9 is a schematic view of a down sampling method, and shows an example of down sampling, and FIG. 11 is a graph of an information extract method using a data relation map.

FIG. 12 is a schematic view of an algorithm structure of a biometric information analysis artificial intelligence-based deep learning method according to an exemplary embodiment of the present invention, FIG. 13 is a schematic diagram of the algorithm feature extraction portion, FIG. 14 is a layer configuration diagram of the feature extraction portion, and FIG. 15 is a schematic diagram of an algorithm blood glucose estimation portion.

FIG. 16 is a schematic diagram of an ensemble algorithm schematic diagram.

FIG. 17 is a flowchart of a biometric information analysis artificial intelligence-based deep learning method according to an exemplary embodiment of the present invention.

**Mode for Invention**

Hereinafter, an analyte analysis method, an apparatus, and a system utilizing an artificial neural network deep learning-based method according to an exemplary embodiment of the present invention will be described.

In the present specification, an analyte analysis method, an apparatus, and a system utilizing an artificial neural network deep learning-based method according to an exemplary embodiment of the present invention are only examples for description of the present invention, and the scope of the present invention is not limited by the exemplary embodiment.

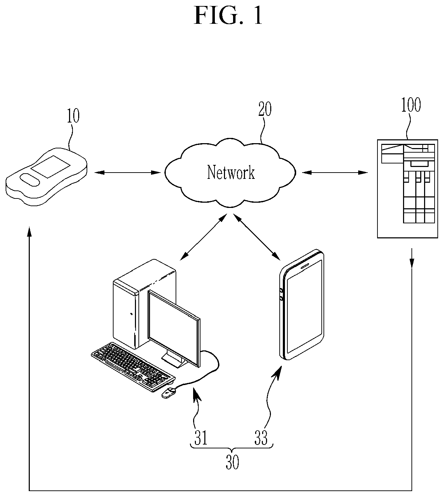

FIG. 1 is a schematic view of an analyte analysis system utilizing an artificial neural network deep learning method according to an exemplary embodiment of the present invention.

As shown in FIG. 1, an analyte analysis system 1 that utilizes an artificial neural network deep learning-based method according to an exemplary embodiment of the present invention includes a biometric information measurer 10 provided with a pair of electrodes, which is the minimum number, a biometric information analysis artificial intelligence-based deep learning server 100 that provides an optimized algorithm for measuring biometric information by using an artificial intelligence-based deep learning technology, a network 20 that connects the biometric information measurer 10 and the artificial intelligence-based deep learning server 100 by wire or wirelessly, and a user terminal 30 that stores and registers biometric information measured by the biometric information measurer 10 or assists learning of the biometric information analysis artificial intelligence-based deep learning server 100, such as a computer, a smart terminal, and the like.

Here, the network 20 is a network that enables transmission and reception of information between the biometric information measurer 10, a personal computer 31, a smart terminal 33, and the biometric information analysis artificial intelligence-based deep learning server 100, such as a public telephone network, an Internet network, and the like, and various types of networks can be used depending on the field to which the present invention is applied, and this belongs to the range of the present invention.

The biometric information measurer 10 is equipped with a modem that can use a backbone communication network such as 2G, 3G, LTE, Wi-Fi, and the like, and thus when a user measures biometric information, the biometric information measurer 10 generates a biometric information message that includes an identifier and measured biometric information and transmits the generated biometric information to the biometric information analysis artificial intelligence-based deep learning server 100.

When the biometric information measurer 10 cannot communicate, a biometric information message that includes an identifier of the biometric information measurer 10 and measured biometric information can be transmitted to the biometric information analysis artificial intelligence-based deep learning server 100 through the user terminal 30 such as the personal computer 31 or the smart terminal 33.

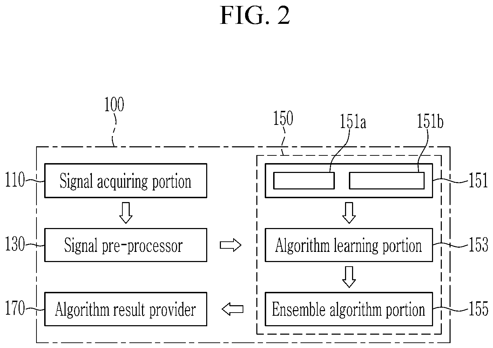

FIG. 2 is a schematic diagram of the biometric information analysis artificial intelligence-based deep learning server in an analyte analysis learning system that utilizes the artificial neural network deep learning-based method according to the exemplary embodiment of the present invention.

As shown in FIG. 2, the biometric information analysis artificial intelligence-based deep learning server 100 may include a signal acquiring portion 110 that acquires one-dimensional time series data through an electrochemical reaction, which occurs from injection of blood collected by the biometric information measurer 10 into a sensor (i.e., a strip), a signal processor 130 that excludes an abnormal signal due to abnormality in blood injection or hardware malfunctioning from the signal acquired by the signal acquiring portion 110, or pre-processes the signal to acquire an optimized biometric information measurement algorithm, which can be used by the biometric information measurer 10, a biometric information measurement algorithm generator 150 that automatically extracts features by utilizing an optimized biometric information measurement algorithm with a deep learning artificial neural network method from the signal processed through the signal processor 130, and an optimized algorithm result provider 170 that can be used in the biometric information measurer 10.

The signal processor 130 converts data to constant scales or distribution through normalization or standardization such that that data can be learned by the biometric information measurement algorithm generator 150, and the converted data may be imaged by combining multichannel data or using signal processing and data conversion such as domain conversion (e.g., time or frequency domain).

The biometric information measurement algorithm generator 150 may include an algorithm structure portion 151 that forms an algorithm structure for blood glucose measurement, an algorithm learning portion 153 that adjusts variables in the algorithm to most accurately predict the blood glucose value where the blood glucose prediction value is true, and an ensemble algorithm portion 155 that calculates a final prediction value by combining at least one of algorithms for improvement of accuracy and the degree of precision in blood glucose value prediction.

In addition, the algorithm structure portion 151 may include a feature extraction portion 151a that extracts features of an analyte included in biometric information signal data which has been pre-processed through the signal processor 130, and a blood glucose value prediction portion 151b that estimates a blood glucose value by using the features acquired by the feature extraction portion 151a.

The algorithm structure portion 151 automatically extracts a feature that reflects a result value which is desired to be classified or measured from an image where peripheral environments such as components of the analyte, a hematocrit, a temperature, and characteristics of interference species are reflected.

The algorithm learning portion 153 draws a weight and a bias between artificial neural network layers, which minimize an error of the result value through the algorithm learning process by using the extracted feature.

The algorithm structure portion 151 can be utilized as a regression model that estimates a desired specific value with the artificial neural network algorithm, and also can be utilized as a classifier that classifies the type of the analyte.

In the present specification, correction of a measurement error due to hematocrit in the blood glucose measurement is described as a preferable exemplary embodiment, but, like glucose testing, measurement values of concentrations of various metabolic materials, for example, organic materials or minerals such as beta-hydroxybutyrate (aka ketone), cholesterol, lactate, creatinine, hydrogen peroxide, alcohols, amino acids, glutamate, and the like can be corrected by applying a specific enzyme. Therefore, the present invention can be used to quantify various metabolic materials by different types of enzymes included in the sample layer composition.

For example, quantification of beta-hydroxybutyrate, glucose, glutamate, cholesterol, lactate, ascorbic acid, alcohol, and bilirubin can be carried out by using β-hydroxybutyrate dehydrogenase, glucose oxidase (GOx), glucose dehydrogenase (GDH), glutamate oxidase, glutamate dehydrogenase, cholesterol oxidase, ascorbic oxidase, alcohol oxidase, alcohol dehydrogenase bilirubin oxidase, and the like.

In the biometric information measurer 10 according to the exemplary embodiment of the present invention, an operation electrode and an auxiliary electrode are provided to face each other on different planes, and a face-to-face type of electrochemical biosensor coated with a reagent composition that includes an enzyme according to a material on the operation electrode and an electron transmission medium may be applied.

Alternatively, in the biometric information measurer 10 according to the exemplary embodiment of the present invention, the operation electrode and the auxiliary electrode are provided on one plane, and a flat electrochemical biosensor coated with a reagent composition that includes an enzyme according to a material on the operation electrode and an electron transmission medium may be applied.

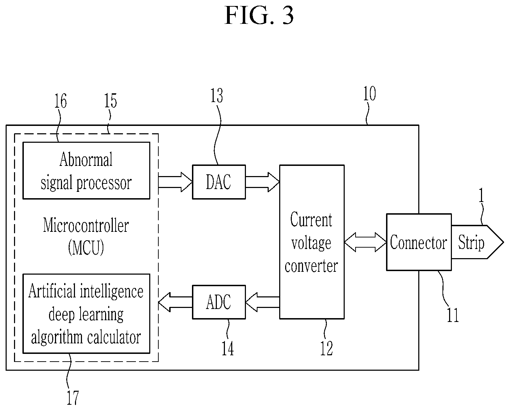

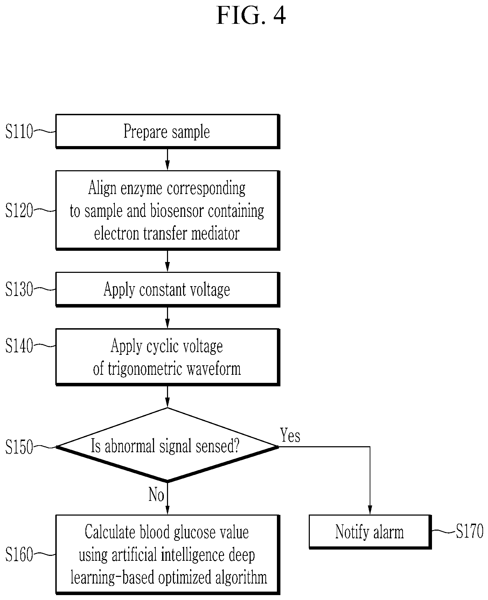

Referring to FIG. 3 and FIG. 4, a blood glucose measurement method utilizing the artificial neural network deep learning-based method according to the exemplary embodiment of the present invention, and an apparatus using the same, will be described.

FIG. 3 is an internal schematic diagram of a blood glucose measurement apparatus utilizing the artificial neural network deep learning-based method according to the exemplary embodiment of the present invention.

As shown in FIG. 3, when a circulating voltage of a triangular wave, a square wave, a stepped wave, or other modified trigonometric waveform is applied after applying a constant voltage, the blood glucose measurement apparatus 10 using the artificial neural network deep learning strategy according to the embodiment of the present invention can provide blood glucose measurement values according to an optimized artificial intelligence-based deep learning algorithm that can improve accuracy and precision of an algorithm, and interference correction performance including hematocrit of the algorithm according to the analyte analysis method utilizing the artificial neural network deep learning strategy according to the embodiment of the present invention, and depart from the existing multiple linear regression method, while maintaining the structure of a pair of operation electrodes and auxiliary electrodes of an existing electrochemical biosensor, that is, the strip 1.

The blood glucose measurement apparatus 10 utilizing the artificial neural network deep learning-based method according to the exemplary embodiment of the present invention effectively carries out calculation of a blood glucose estimation artificial intelligence-based deep learning algorithm, which is optimized even in limited hardware such that operation time can be taken within 8 seconds.

In the blood glucose measurement apparatus 10 utilizing the artificial neural network deep learning-based method according to the exemplary embodiment of the present invention, when the electrochemical biosensor 1 is embedded in a connector 11, the connector 11 is electrically connected to a current-voltage converter 12, and a microcontroller unit (MCU) 15 applies a constant voltage through a digital-to-analog converter (DAC) circuit 13 and applies a cyclic voltage of a triangular waveform to the operation electrode of the strip 1.

For this, firmware of the blood glucose measurement apparatus 10 utilizing the artificial neural network deep learning-based method according to the exemplary embodiment of the present invention stores a constant, which may generate a predetermined cyclic voltage of a triangular waveform in a memory, records a predetermined constant in a register of the DAC 13 when a constant voltage is applied, and records constants stored in the memory by increasing or decreasing with a predetermined time period in the register of the DAC 13 when the cyclic voltage of the triangular waveform is applied. However, the waveform of the applied cyclic voltage is just an example, and cyclic voltages of all waveforms, which are known in the art, are included.

The MCU 15 applies a corresponding voltage between the two electrodes of the strip according to the constant value recorded in the register of the DAC 13.

As shown in FIG. 3, when a constant voltage is applied and a cyclic voltage of a triangular waveform is applied, a response current measured through the strip 1 may be directly measured through an ADC 14 through the connector 11 and the current-voltage converter 12.

Meanwhile, the blood glucose measurement apparatus 10 utilizing the artificial neural network deep learning-based method according to the exemplary embodiment of the present invention further includes an abnormal signal processor 16 and an artificial intelligence deep learning algorithm calculator 17, and when an abnormal signal due to an abnormality in blood injection or hardware occurs, the abnormal signal processor 16 informs the abnormality by displaying in a display, to thereby prevent the artificial intelligence deep learning algorithm calculator 17 from performing unnecessary calculation.

The artificial intelligence deep learning algorithm calculator 17 can acquire a blood glucose measurement value within 8 seconds from the response current measured through the strip 1 with an optimized artificial intelligence deep learning blood glucose measurement algorithm drawn by the biometric information analysis artificial intelligence-based deep learning server 100.

Hereinafter, referring to FIG. 4, a blood glucose measurement method utilizing the artificial neural network deep learning-based method according to the exemplary embodiment of the present invention will be described.

FIG. 4 is a flowchart of a blood glucose measurement method utilizing the artificial neural network deep learning-based method according to the exemplary embodiment of the present invention.

Referring to FIG. 4, a blood glucose measurement method utilizing the artificial neural network deep learning-based method according to the exemplary embodiment of the present invention includes: preparing a sample (S110); loading an electrochemical face-to-face type of biosensor 1 to a biometric information measurer 10 (S120); applying a constant voltage through the current voltage converter 12 at the moment that the biosensor 1 contacts the sample (blood) and thus the blood is wetted with the operation electrode and auxiliary electrode of the electrochemical biosensor 1 (S130); and continuously applying a cyclic voltage of a triangular waveform at a chronical voltage application termination point after the application of the constant voltage (S140).

The abnormal signal processor 16 determines whether or not an induced current is an abnormal signal (S150), and when the induced current is the abnormal signal, the abnormal signal processor 16 notifies the abnormality by displaying and the like on a display (S151).

When it is not determined to be an abnormal signal, a blood glucose measurement can be acquired from an induced current measured using an artificial intelligence deep learning blood glucose measurement algorithm by the artificial intelligence deep learning algorithm calculator 17.

Comparative Example

Like the blood glucose measurement method utilizing the artificial neural network deep learning-based method according to the exemplary embodiment of the present invention, a blood glucose measurement method using an existing multiple regression method acquired concentration of blood glucose by applying a constant voltage and a triangular waveform to a conventional planar-shape or face-to-face type of electrochemical biosensor through a biometric information measurer 10 and measuring a current value generated by applying an oxidation potential of an electron transfer mediator that is reduced in an operation electrode.

First, an existing method (hereinafter referred to as an existing method) that adopts an algorithm that calculates by using a multiple linear regression method with respect to accuracy performance and the proposed method of the present invention were compared as follows.

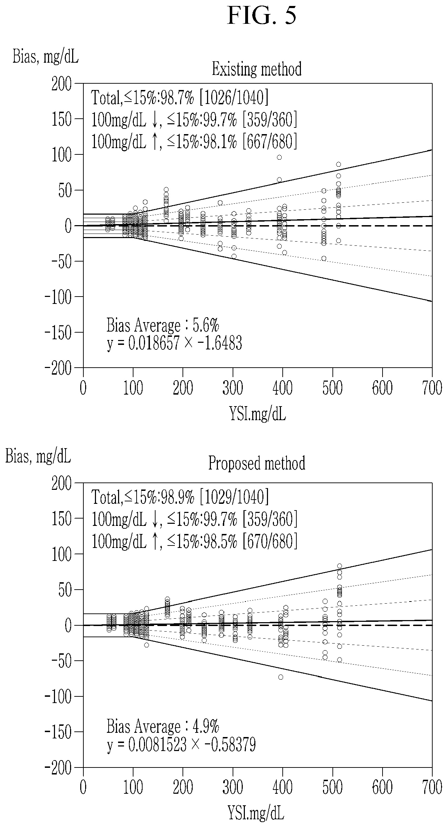

A total of two accuracy performance evaluations were conducted with the analysis values obtained for the same samples in the YSI2300 system, which is a standard analyzer (hereinafter, the standard of comparison of all exemplary embodiments is a comparison with the measured value of YSI2300). Table 1 and FIG. 5 show the existing method and the proposed method, and in the two clinical trials, it was confirmed that the new algorithm result was statistically improved (p=0.047).

For that is, the existing method and the proposed method were applied with respect to data sets 1 and 2, and for 1040 and 600 samples, respectively, and measurement accuracy within $\pm 15$ mg/dL compared to the reference value was measured for a case that the blood glucose concentration based on ISO15197 was 100 mg/dL or higher and a case that the blood glucose concentration of less than 100 mg/dL was within $\pm 15$ % of the reference value. Bias for the measured value of the standard analyzer is calculated to be (blood glucose estimated value-reference value) when the blood glucose concentration is less than 100 mg/dL, and ((blood glucose estimated value-reference value)/standard Value)*100 when the blood glucose concentration is 100 mg/dL or more. Accuracy, a bias average, and an average root mean square bias (RMSB) were used as indicators of accuracy performance. RMSB is calculated to be

$$RMSB = \sqrt{\left(\frac{1}{n} \sum_{i=1}^{n} bias_i\right)^2}.$$

Table 1 shows a bias comparison with respect to a blood glucose measured value obtained from a system using the multiple linear regression method of the existing method and measured values obtained from the standard analyzer YSI2300 system for blood glucose measurement values obtained from a system employing a deep learning algorithm according to the exemplary embodiment of the present invention.

(Table 1)

| Test | Algorithm type | Number of samples | Accuracy within bias (15 mg/dL or %) | | | Bias average | RMBS |
|------|----------------|-------------------|------------------|--------------------|---------------------|--------------|------|
| | | | 100 mg/dL or less | 100 mg/dL or more | Total concentration | | |
| Primary | existing | 1040 | 99.7 | 98.1 | 98.7 | 5.6 | 11.1 |

(continued)

| Test | Algorithm type | Number of samples | Accuracy within bias (15 mg/dL or %) | | | Bias average | RMBS |
|---|---|---|---|---|---|---|---|
| | | | 100 mg/dL or less | 100 mg/dL or more | Total concentration | | |
| Test | method | | | | | | |
| | Proposed method | 1040 | 99.7 | 98.5 | 98.9 | 4.9 | 9.8 |
| | | | | | | | |
| Secondary Test | existing method | 600 | 99.5 | 97.9 | 98.5 | 5.7 | 13.1 |
| | Proposed method | 600 | 99.5 | 99.7 | 99.7 | 4.4 | 6.7 |
| *Bias average: Bias absolute value average (mg/dL or %) RMBS: Bias root mean square (mg/dL or %) | | | | | | | |

FIG. 5 is a graph that shows clinic test accuracy performance according to the existing method and the proposed method. As shown in FIG. 5 and Table 1, the accuracy of the proposed method was 98.9 % and 99.7 % in the primary and secondary tests, and the accuracy was improved compared to the existing methods at 98.7 % and 98.5 %. In addition, the bias average was improved in the proposed method compared to the existing method, from 5.6 to 4.9 and from 5.7 to 4.4, and the RMSB was improved from 11.1 to 9.8 and from 13.1 to 6.7.

In addition, Table 2 shows the average bias comparison of blood glucose measurement values obtained from a system using the multiple linear regression method of the existing method and blood glucose measurement values obtained from a system employing the deep learning algorithm according to the exemplary embodiment of the present invention with respect to blood glucose measurement values obtained from 42 % hematocrit samples.

As shown in Table 2, hematocrit effect evaluation was performed, and it can be seen that the proposed method shows excellent results compared to the existing method.

The sample is a standard glucose solution, and the standard glucose solution refers to blood prepared by matching blood extracted from a vein to hematocrit at 15 % to 65 % and making the blood have a specific glucose concentration by using a glucose analyzer, that is, 2300 Stat Plus of YSI, and standard blood samples of the prepared concentration were measured 10 times each at 23 degrees.

With reference to the ISO15197 standard, based on the average of 42 % hematocrit of each glucose sample, the evaluation of hematocrit effect should have a result that the average difference between each hematocrit should be 10 mg/dL or less for less than 100 mg/dL of glucose and 10 % or less for 100 mg/dL or more of glucose.

The values shown in Table 2 show an average difference in each hematocrit based on the blood glucose value of the sample of blood glucose of 340 mg/dL and hematocrit of 42 %. The average difference occurs depending on the hematocrit.

That is, it can be seen that the estimated value of blood glucose varies depending on the amount of hematocrit in the blood.

Mean-Absolute-Difference (MAD) was used for quantitative comparison of hematocrit correction ability between the existing method and the proposed method. MAD is an absolute average value of the average difference between each hematocrit based on 42 % hematocrit.

More specifically, in the hematocrit effect evaluation, MAD was 5.9 in 340 mg/dL of glucose in the existing method, but MAD was 3.5 in the proposed method such that hematocrit correction performance was improved.

(Table 2)

| | Glucose (mg/dL) | Hematocrit (%) | | | | | MAD |
|---|---|---|---|---|---|---|---|
| | | 15 | 30 | 42 | 53 | 65 | |
| Convention method | 340 | 6.6 | 4.4 | 0 | -3.5 | -5.8 | 5.9 |
| Proposed method | 340 | -2.6 | 0 | 0 | -1.7 | -5.9 | 3.5 |
| Unit: mg/dL or % Mean Absolute Difference (MAD): average of absolute value differences | | | | | | | |

In addition, accuracy performance evaluation was performed. The accuracy of the algorithm is calculated based on ISO15197, and the blood glucose concentration of less than 100 mg/dL is calculated as the standard deviation of the blood glucose estimate, while the blood glucose concentration of more than 100 mg/dL is calculated as the coefficient of variation (CV). CV is the standard deviation of the blood glucose value of each experimental condition divided by the arithmetic average.

Meanwhile, Table 3 shows accuracy comparison results of blood glucose measurement values obtained from a system using a multiple linear regression method of the existing method and blood glucose measurement values obtained through iterative measurements in a system employing the deep learning algorithm according to the exemplary embodiment of the present invention.

Here, when the glucose is less than 100 mg/dL, the standard deviation value of the measured value is used, and when it is more than 100 mg/dL, the coefficient of variation (CV) of the measured values is used.

As shown in Table 3, in the exemplary embodiment of the present invention, the accuracy of the method for measuring the concentration of a specific material in blood acquired p=0.000 in the F-test, which is a distribution ratio verification compared to a method for measuring the concentration of a specific material in blood according to a comparative example, and thus it was confirmed to be a statistically significant improvement.

(Table 3)

| CV (%) n=50 (per condition) | Glucose Level (mg/dL) | | | | |
|---|---|---|---|---|---|
| | 35 | 94 | 135 | 211 | 327 |
| existing method | 2.2 | 3.4 | 5.0 | 3.6 | 3.5 |
| Proposed method | 1.8 | 2.6 | 3.0 | 2.6 | 2.5 |
| Unit SD, mg/dL (<100 mg/dL) CV, % (≥100 mg/dL) | | | | | |

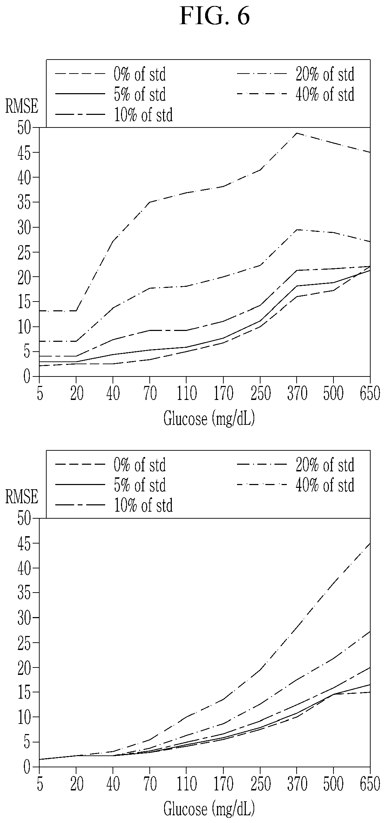

Referring to FIG. 6, noise sensitivity performance will be described.

As shown in FIG. 6, the effect of the algorithm value when the noise of a certain size was randomly applied to the strip reaction signal was confirmed. When random noise of a 0 to 40 % size compared to the standard deviation of the existing signal was added, the change in the algorithm value was confirmed. In the case of 110 mg/dL, which is important in clinical trials, in the existing method, the root-mean-square-error (RMSE) differs by about 5 to 37 mg/dL depending on the noise size, whereas in the proposed method, it is about 4 to 9 mg/dL and thus stability to noise was improved.

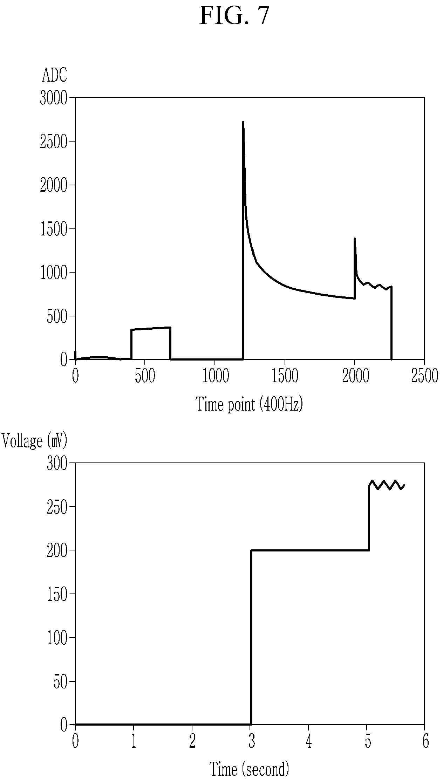

Hereinafter, an analyte analysis learning method utilizing an artificial neural network deep learning-based method will be described in detail with reference to FIG. 7 to FIG. 17.

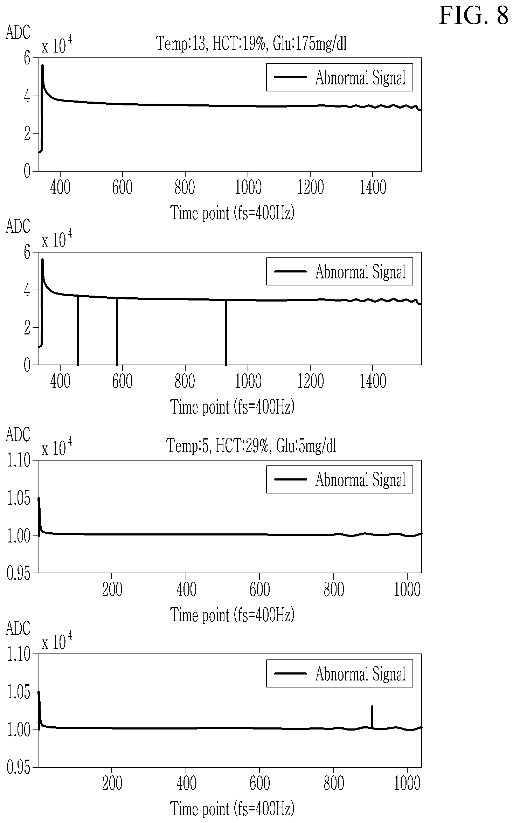

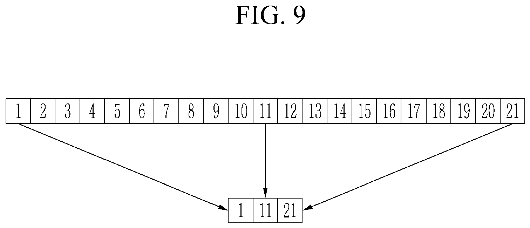

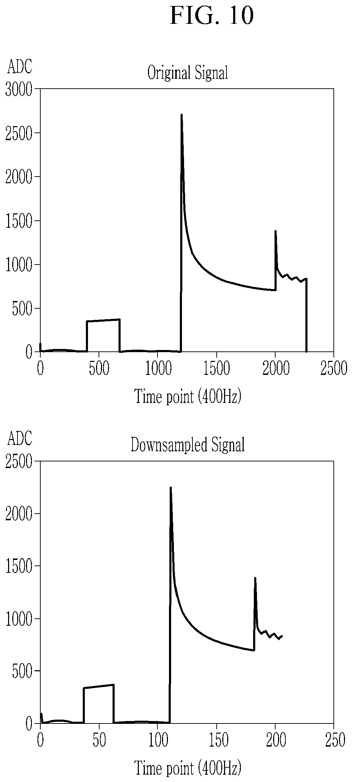

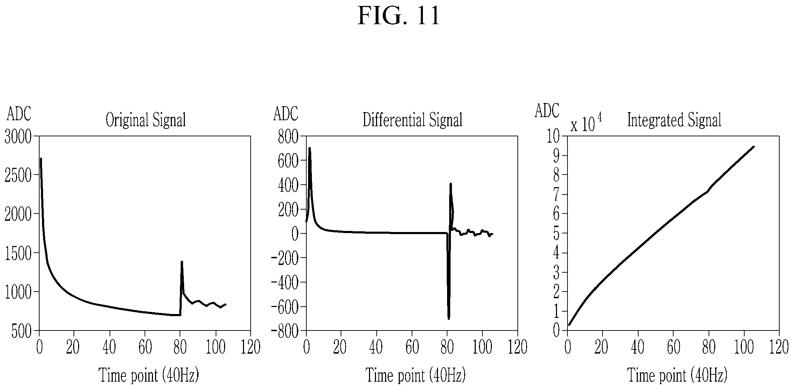

FIG. 7 shows a voltage and a response current applied in a biometric information analysis artificial intelligence-based deep learning method according to an exemplary embodiment of the present invention, FIG. 8 is an exemplary view of an abnormal signal, FIG. 9 is a schematic view of a down sampling method, FIG. 10 shows an example of down sampling, and FIG. 11 is a graph of an information extract method using a data relation map.

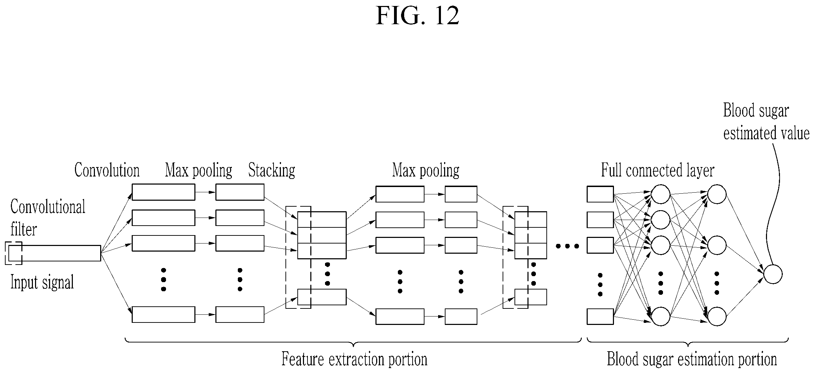

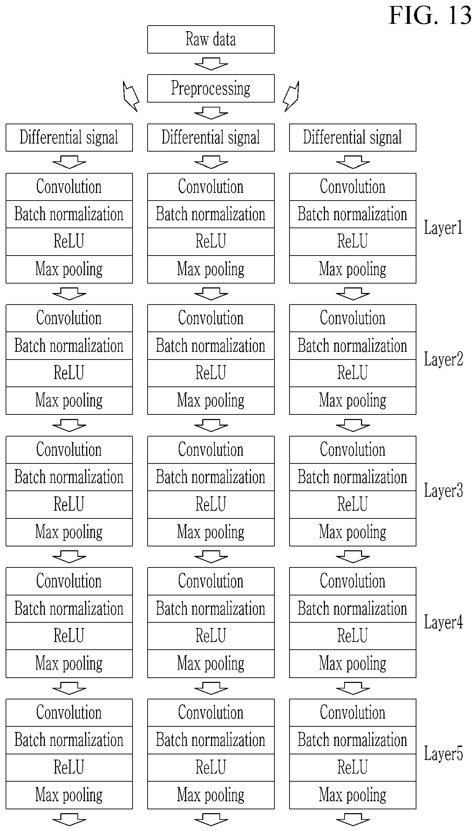

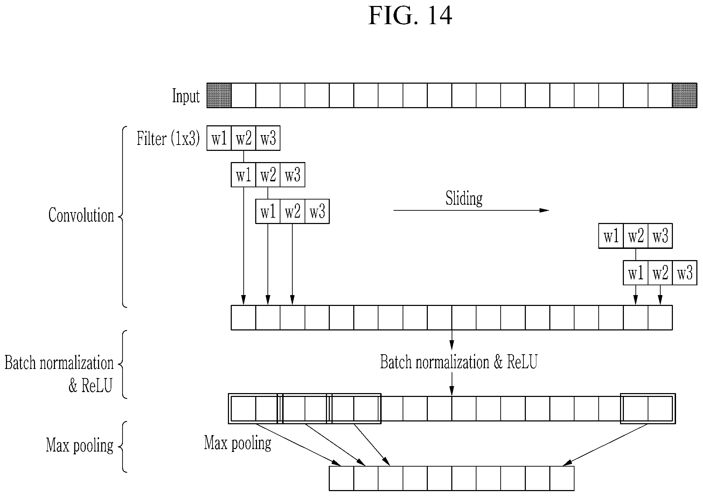

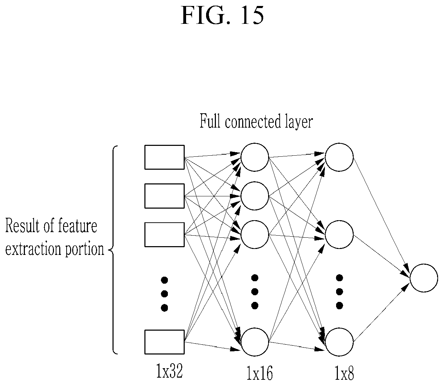

FIG. 12 is a schematic view of an algorithm structure of a biometric information analysis artificial intelligence-based deep learning method according to an exemplary embodiment of the present invention, FIG. 13 is a schematic diagram of the algorithm feature extraction portion, FIG. 14 is a layer configuration diagram of the feature extraction portion, and FIG. 15 is a schematic diagram of an algorithm blood glucose estimation portion.

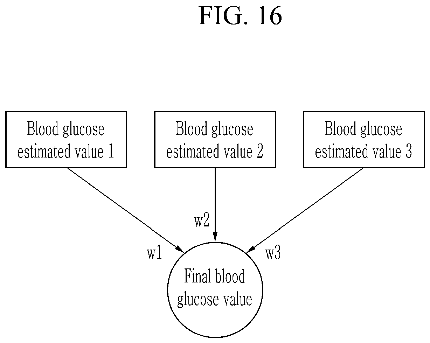

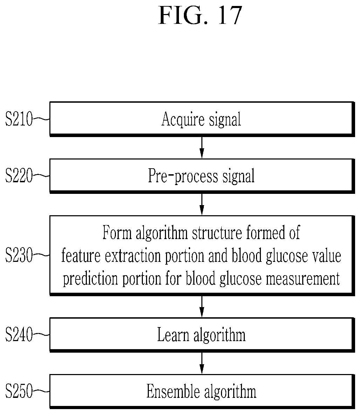

FIG. 16 is a schematic diagram of an ensemble algorithm schematic diagram, and FIG. 17 is a flowchart of a biometric information analysis artificial intelligence-based deep learning method according to an exemplary embodiment of the present invention.

A biometric information analysis artificial intelligence-based deep learning method according to an exemplary embodiment of the present invention acquires one-dimensional time series data through an electrochemical reaction that occurs due to injection of blood collected by the biometric information measurer 10 into a strip in the signal acquiring portion 110 (S210).

Experimental data were obtained under the variables and experimental conditions as shown in Table 4 in a chamber where temperature and humidity can be controlled under various experimental conditions of 5 to 50 °C, hematocrit of 10 to 70 %, and glucose of 10 to 630 mg/dL.

Table 4 shows experimental conditions for acquiring learning data.

(Table 4)

| Variable | Experimental condition |
|---|---|
| Temperature | 5, 9, 13, 18, 23, 28, 34, 42, 50 (°C) |
| Glucose | 10, 35, 70, 120, 180, 250, 330, 420, 520, 630 (mg/dL) |
| Hematocrit | 10, 20, 30, 42, 49, 56, 70 (%) |

As blood for acquiring experimental data, blood prepared under the above experimental conditions was used. A method for preparing blood uses venous blood collected in an anticoagulant tube and centrifuged to separate it into plasma and blood cells. The hematocrit was adjusted by adjusting a mixing ratio of plasma and blood cells, and glucose of desired concentration was added such that blood for each experimental condition was prepared.

In a used strip, a pair of electrodes (operation electrode and auxiliary electrode) where reagents containing enzymes, electron transfer mediators, various stabilizers, and distribution agents are coated to a sample cell of a capillary structure are disposed.

When a method of acquiring 400 pieces of data per second in a strip having two electrodes to acquire a single blood sample, collection of a total of 2260 pieces of data is required for the sequence voltage applied for 2.65 seconds to measure 3 seconds of incubation (time for waiting for chemical reaction to occur between a strip enzyme and blood) and reaction. For example, data acquired from an applied sequence and a blood reaction is as shown in the graph of FIG. 7.

In order to acquire the data necessary for learning, a plurality of researchers need to work in a chamber capable of constant temperature and humidity for a considerable period of time. In the exemplary embodiment, a combination of temperature, glucose, and hematocrit experimental conditions has been collected to obtain over 100 million pieces of data in total through repeated measurements.

The signal processor 130 converts the data obtained through repeated measurement by combining the temperature, glucose, and hematocrit experimental conditions obtained from the signal acquiring portion 110 to a certain size (scale) or distribution through normalization or standardization.

The converted data is imaged by combining multi-channel data or using signal processing and data conversion such as domain conversion (e.g., time or frequency domain). When acquiring a signal, abnormal data may be generated due to a blood injection abnormality and a hardware abnormality, and for example, when a noise spike occurs as an abnormal signal, the signal processor 130 processes it as an error through an algorithm that excludes abnormal signals.

In order to process abnormal signals as errors, an extraction method when the degree of data change over time becomes more than a certain value and an extraction method when it is out of the signal range that can occur in the normal signal are used were mixed and used.

Meanwhile, data from an electrochemical reaction signal acquired from an electrode was collected 1060 times by applying a specific sequence for 2.65 seconds by using a method for collecting 400 pieces of data per second, and in case of the biometric information measurer 10 of the present invention, a problem that it takes about 32 seconds occurs in order to process all 1060 pieces of data in limited hardware such as STM32L MCU in the case of the algorithm structure of the exemplary embodiment.

When time spent for blood glucose measurement is 32 seconds, marketability is deteriorated such that optimization is required. Thus, a row rank approximation method and a down sampling method are applied to reduce dimensions of a source signal formed of 1060 pieces of data to thereby process an algorithm with a lot of operations in limited hardware.

In the biometric information analysis artificial intelligence-based deep learning method according to the exemplary embodiment of the present invention, as shown in FIG. 9, the signal processor 130 performed a down sampling method that can maintain features of a time series signal while reducing the amount of operations of the algorithm to a level of a tenth part as signal preprocessing.

As shown in FIG. 10, although the data size was reduced to a tenth level, a signal having the characteristics of the existing time series signal was acquired. The time taken for blood glucose measurement was reduced from about 32 seconds to 3 seconds as the down sampling result.

As shown in FIG. 11, in the biometric information analysis artificial intelligence-based deep learning method of the exemplary embodiment of the present invention, the signal processor 130 applied a time-based data relation map and domain change to acquire additional information from a source signal of a blood sample acquired from a single electrode.

In the biometric information analysis artificial intelligence-based deep learning method according to the exemplary embodiment of the present invention, the source signal is converted into differential and integral signals and then added. Thus, an input signal is formed of the source signal acquired from the strip, a differential signal of the source signal, and an integral signal of the source signal. Through this, an effect of obtaining a signal from a multielectrode can be provided through a signal obtained from a single electrode.

FIG. 12 is an algorithm structure schematic view of the biometric information analysis artificial intelligence-based deep learning method according to the exemplary embodiment of the present invention, FIG. 13 is a schematic diagram of the algorithm feature extraction portion, FIG. 14 is a layer configuration diagram of the feature extraction portion, FIG. 15 is a schematic diagram of the algorithm blood glucose estimation portion, and FIG. 16 is a schematic view of an ensemble algorithm.

Meanwhile, as shown in FIG. 12, in the biometric information analysis artificial intelligence-based deep learning method according to the exemplary embodiment of the present invention, the algorithm structure portion 150 for blood glucose measurement forms an algorithm structure for blood glucose measurement (S230).

The algorithm structure for blood glucose measurement is divided into a feature extraction portion 151 and a blood glucose value prediction portion 153. As the algorithm size is increased, computing power is increased, while causing an increase of resource consumption. In order to implement an algorithm with a large amount of operations, such as deep learning, in limited hardware such as a blood glucose measurement device, an algorithm structure needs to be minimized. The purpose is not to simply reduce the size of the algorithm, but to reduce the algorithm structure while minimizing performance degradation.

In order to minimize the algorithm structure, connection pruning that removes an unimportant connection (weight value), weight encoding-sharing that groups similar connections (weight values), and an algorithm model normalization technology are applied to minimize the algorithm structure while minimizing performance degradation of the algorithm.

The input signal of the feature extraction portion 151 is 1x106 size data that has been processed through the above-described signal preprocessing. In order to extract features included in the data, a five-layered structure was constructed.

The reason that the feature extraction portion 151 is formed of multiple layers is to embody abstract features that can be obtained from raw data by processing through multiple layers. In each layer of the feature extraction portion 151, a convolution filter for extracting features is set.

In the biometric information analysis artificial intelligence-based deep learning method according to the exemplary embodiment of the present invention, depending on the input signal, a filter of 1x5 for the original signal and a filter of 1x3 for the differential and integral signals were used, and the number of filters used for each layer is as shown in Table 5.

Table 5 shows a filter size and the number of filters used in the exemplary embodiment.

(Table 5)

|  | Filter size | | | Number of filters | | |
|---|---|---|---|---|---|---|
|  | Source signal | Differential signal | Integral signal | Source signal | Differential signal | Integral signal |
| Layer 1 | 5x1 | 3x1 | 3x1 | 16 | 16 | 16 |
| Layer 2 | 5x1 | 3x1 | 3x1 | 16 | 16 | 16 |
| Layer 3 | 5x1 | 3x1 | 3x1 | 16 | 16 | 16 |
| Layer 4 | 5x1 | 3x1 | 3x1 | 32 | 32 | 32 |
| Layer 5 | 5x1 | 3x1 | 3x1 | 32 | 32 | 32 |

Each layer is formed of a part that performs convolution, a part that performs batch normalization, an activation function, and max pooling.

Convolution

Convolution is to sequentially perform an operation from the start to the end of the data by using filters set as shown in FIG. 13. The convolution operation is $Conv_{out} = \sum_{i=1}^{n} input_i * filter_i$, and n denotes a filter size.

[Batch normalization and ReLU]

Batch normalization and ReLU were used to solve a covariate shift and gradient vanishing, which may cause a problem related to deep learning.

Batch normalization is calculated by the following formula for each dimension when the data has k dimensions.

$$\hat{x} = \frac{X^k - E[X^k]}{\sqrt{Var(x^k)}}$$

The ReLU function is calculated by the following formula for batch normalization.

$$F(x) = \max(0, \hat{x})$$

[Pooling]

In order to reduce the size of data derived through the convolution operation, max pooling and mean pooling were applied in the feature extraction portion.

Each layer is formed of a portion performing convolution, a portion performing batch normalization, an activation function, and max pooling or mean pooling.

FIG. 14 shows an example of each layer of the feature extraction portion formed of five layers. The algorithm feature extraction portion outputs 32 features according to each signal.

The blood glucose value prediction portion 153 estimates a blood glucose value using 32 features obtained using the feature extraction portion 151. The blood glucose value prediction portion 153 is formed of a total of two layers. 32 values are reduced to 16 using a 32x16 weight value array in the first layer, 16 features are reduced to 8 using a 16x8 weight value array in the second layer, and then a final blood glucose value is estimated.

Each feature is called a node, and nodes of neighboring layers are connected with each other. Through learning, weight values of the nodes are determined.

As shown in FIG. 15, the algorithm learning portion 153 learns a blood glucose measurement algorithm.

In this case, variables in the algorithm are adjusted to most accurately predict a blood glucose value of which the blood glucose prediction value is the true value. The variable in the algorithm may be a filter value of the feature extraction portion 151 or a weight value between nodes of the blood glucose value prediction portion 153.

The goal of optimization is to minimize a loss function. The loss function was defined as the root mean square error value of the blood glucose value and the predicted value.

As a method for optimizing the loss, a method showing the maximum performance was applied by using a gradient descent optimizer, a momentum optimization, a Nesterov accelerated gradient, AdaGrad, RMSProp, and Adam optimization.

Adam optimization was used to update the variables in the algorithm according to a difference between the blood glucose prediction value and the true value, and the degree of variable update was determined by a learning rate ($\eta$) and was set to 0.00001 in this algorithm. The basic loss optimization process is as follows.

1. Set variables and a learning rate to be optimized.
2. Repeat the study until a difference between the predicted value of blood glucose and the true value becomes minimal.

1) Randomly select a part of the learning data when learning is repeated.

2) Calculate the degree of compensation for the difference between the predicted and true values for the selected data.

3) Update the variables to be optimized in the algorithm by the learning rate according to the calculated compensation degree.

When the minimum data required for the operation are accumulated, the operation is performed separately from the data collection to reduce the algorithm operation time. That is, it is not a method of operating data after collecting all data, but a method of simultaneously collecting and operating data.

In the present exemplary embodiment, operation time of about 3.3 seconds is required for operation after data collection, but the operation time is improved by about 2.3 seconds by allowing all operations to be completed within about 1 second after data collection through the above-mentioned method.

Next, an ensemble algorithm portion 155 combines at least one algorithm to improve the accuracy and precision of blood glucose value prediction to calculate the final prediction value.

As shown in FIG. 16, when the input signal is three signals, i.e., a source signal, a differential signal, and an integral signal, a blood glucose value for each signal is predicted, and the final blood glucose value is calculated through a weighted average of the blood glucose values obtained by three algorithms.

The final value calculation method can be assigned different weight values depending on the importance of each algorithm, and a small network algorithm can be additionally constructed using the output values of the three algorithms.

Referring to FIG. 17, a flowchart of the biometric information analysis artificial intelligence-based deep learning method according to the exemplary embodiment of the present invention will be simply described again.

A biometric information analysis artificial intelligence-based deep learning method according to another aspect of the present invention may include: acquiring a signal through an electrochemical reaction that occurs due to injection of blood collected through a biometric information measurer into a strip having a pair of electrodes (S210); processing the acquired signal to a signal for artificial intelligence deep learning (S230); forming an algorithm structure formed of a feature extraction portion and a blood glucose value prediction portion of the signal for blood glucose measurement (S240); learning a blood glucose measurement algorithm by adjusting variables in the algorithm to predict the most accurate blood glucose value of which the blood glucose prediction value has the true value (S250); and performing optimization (S260).

**Industrial Applicability**

According an analyte analysis method utilizing an artificial neural network deep learning-based method, an apparatus, and a system can improve the accuracy, precision, and interference correction performance including hematocrit of an algorithm, unlike an existing multiple linear regression method, and provides an optimized blood glucose estimation artificial intelligence-based deep learning technology that can be implemented with meter hardware with a limited structure and a sensor strip.

**Claims**

1. An analyte analysis system utilizing an artificial neural network deep learning-based method, comprising
   a biometric information measurer, and
   a biometric information analysis artificial intelligence-based deep learning server that includes: a signal acquiring portion acquiring a signal through an electrochemical reaction, which occurs due to injection of blood collected by the biometric information measurer into a strip that includes a pair of electrodes; a signal processor pre-processing the signal acquired by the signal acquiring portion as a signal for artificial intelligence deep learning; a biometric information measurement algorithm generator that automatically extracts features by using an artificial neural network deep learning-based method for a biometric information measurement algorithm optimized using the signal having been processed through the signal processor; and an optimized algorithm result providing portion that provides the optimized biometric information measurement algorithm to the biometric information measurement apparatus.

2. The analyte analysis system utilizing the artificial neural network deep learning-based method of claim 1, wherein the signal processor excludes a signal from an abnormality in blood injection or an abnormality in hardware from among signals acquired by the signal acquiring portion, the biometric information measurement algorithm generator comprises an algorithm structure portion that forms an algorithm structure for blood glucose measurement, and an algorithm learning portion adjusts variables in the algorithm to a most accurate measure of a blood glucose value of which a blood glucose prediction value is a true value, wherein an ensemble algorithm portion calculates a final prediction value by combining one or more of algorithms for improvement of accuracy and precision in blood value prediction.

3. The analyte analysis system utilizing the artificial neural network deep learning-based method of claim 1, wherein the algorithm structure portion comprises a feature extraction portion that extracts features of an analyte included in biometric information signal data which has been signal-preprocessed by the signal processor, and a blood glucose value prediction portion that estimates a blood glucose value by using features acquired by using the feature extraction portion.

4. The analyte analysis system utilizing the artificial neural network deep learning-based method of claim 1, wherein the algorithm structure portion automatically extracts features that reflect a result value desired to be classified or measured from an image that reflects properties such as components of the analyte, a hematocrit, a temperature, and characteristics of interference species by utilizing a deep learning artificial neural network method.

**5.** The analyte analysis system utilizing the artificial neural network deep learning-based method of claim 1, wherein the algorithm learning portion derives a variable value in the artificial neural network algorithm where an error of a predicted result value is minimized through an algorithm learning process using the extracted features.

**6.** A blood glucose measurement apparatus utilizing an artificial neural network deep learning-based method, comprising:

a connector equipped with an electrochemical biosensor having a pair of electrodes;
a current-voltage converter that is electrically connected with the connector;
a digital-analog converter circuit that is controlled to apply a constant voltage to the pair of electrodes of the electrochemical biosensor, and a cyclic voltage having a triangular waveform, a square waveform, a staircase waveform, or other modified trigonometric function waveform; and
a microcontroller that controls the connector and the digital-analog converter,
wherein the microcontroller comprises an artificial intelligence deep learning algorithm calculator that automatically calculate blood glucose according to an optimized artificial intelligence-based deep learning algorithm acquired from the analyte analysis system utilizing the artificial neural network deep learning-based method according to any one of claim 1 to claim 5.

**7.** The blood glucose measurement apparatus utilizing the artificial neural network deep learning-based method of claim 6, further comprising an abnormal signal processor that warns of an error or abnormality by displaying the same in a display when a connection error occurs in the electrochemical biosensor and the connector or when an abnormal signal is detected due to an abnormality in blood injection or hardware.

**8.** The blood glucose measurement apparatus utilizing the artificial neural network deep learning-based method of claim 6, wherein
the artificial intelligence deep learning algorithm calculator acquires a blood glucose measurement value within 8 seconds from a response current measured through an electrochemical biosensor with an optimized artificial intelligence deep learning blood glucose measurement algorithm derived by a biometric information analysis artificial intelligence-based deep learning server.

**9.** A blood glucose measurement method utilizing an artificial neural network deep learning-based method, comprising:

preparing a sample;
loading an electrochemical biosensor into a biometric information measurer;
applying a constant voltage when the biosensor contacts the sample and thus blood wets an operation electrode and an auxiliary electrode of the electrochemical biosensor, and continuously applying a cyclic voltage having a triangular waveform, a square waveform, a staircase waveform, or other modified trigonometric function waveform at a chronical voltage application termination point after the application of the constant voltage; and
calculating a blood glucose value measured value from a response current measured by an optimized artificial intelligence deep learning blood glucose measurement algorithm acquired from the analyte analysis system utilizing the artificial neural network deep learning-based method of any one of claim 1 to claim 6.

**10.** The blood glucose measurement method utilizing an artificial neural network deep learning-based method of claim 9, comprising:

determining whether the response current is an abnormal signal; and
warning and displaying of an abnormality without calculating a blood glucose value when the response current is determined as an abnormal signal.

**11.** A biometric information analysis artificial intelligence-based deep learning method comprising:

acquiring a signal through an electrochemical reaction that occurs due to injection of blood collected through a biometric information measurer to a strip having a pair of electrodes;
processing the acquired signal as a signal for artificial intelligence deep learning;
forming an algorithm structure formed of a signal feature extraction portion and a blood glucose prediction portion for blood glucose measurement;
learning a blood glucose measurement algorithm by adjusting all variables in the algorithm to minimize a difference between a blood glucose prediction value and a true value; and

using an ensemble algorithm.

12. The biometric information analysis artificial intelligence-based deep learning method of claim 11, wherein the signal acquiring portion acquires a signal while changing a temperature within a range of 5 to 50 °C, hematocrit within a range of 10 to 70 %, and glucose within a range of 10 to 630 mg/dL, and applies a specific voltage to a pair of electrodes of the strip within 3 seconds to measure a reaction with incubation for 3 seconds by using a method acquiring 400 pieces of data per second.

13. The biometric information analysis artificial intelligence-based deep learning method of claim 11, wherein the signal processing comprises processing an abnormal signal due to an abnormality in blood injection and an abnormality in hardware in a signal acquisition, and
the processing of the abnormal signal comprises, when a noise spike occurs, excluding the noise spike using an abnormal signal exclusion algorithm, or excluding it when a difference between an abnormal signal and data exceeds a predetermined value.

14. The biometric information analysis artificial intelligence-based deep learning method of claim 11, wherein the signal processing comprises using down sampling below 80 Hz to reduce an algorithm operation amount of the blood glucose measurement apparatus and changing a relationship map or domain to acquire additional information from an original signal of a blood sample obtained from a single electrode, and as the relationship map of data over time, the original signal, a differential signal, and an integral signal are used.

15. The biometric information analysis artificial intelligence-based deep learning method of claim 11, wherein an input signal of the feature extraction portion is signal-processed data of a 1x106 size, includes 5 layers for extraction of features included in the data, and depending on the input signal, a 1x5 sized filter is used for an original signal and a 1x3 size filter is used for a differential signal and an integral signal, and each layer is formed of a portion that performs convolution, a portion that performs batch normalization, an activation function, and pooling to extract 32 features according to each signal.

16. The biometric information analysis artificial intelligence-based deep learning method of claim 11, wherein the blood glucose value prediction portion estimates a blood glucose value by using 32 features obtained by the feature extraction portion, and the blood glucose value prediction portion is formed of two layers and reduces the 32 features to 16 features by using a 32x16 weight value array in a first layer, reduces the 16 features to 8 features by using a 16x8 weight value array in the second layer, and then finally estimates a blood glucose value.

17. The biometric information analysis artificial intelligence-based deep learning method of claim 11, wherein the algorithm learning comprises: setting a variable and learning rate to be optimized; and repeating a process of learning of optimizing a variable for the algorithm until a difference between a blood glucose prediction value and the true value becomes minimal and updating the degree of compensation for the difference between the predicted and true values by as much as the learning rate.

18. The biometric information analysis artificial intelligence-based deep learning method of claim 11, wherein the using of the ensemble algorithm comprises calculating a final blood glucose value through a weighted sum of the blood glucose values obtained by the algorithm when the input signal is three signals, which are an original signal, a differential signal, and an integral signal.

# FIG. 1

# FIG. 2

100

150   151a   151b

| 110 — | Signal acquiring portion | | 151a | 151b | — 151 |

110 — Signal acquiring portion

⇩

130 — Signal pre-processor   ⇒   Algorithm learning portion — 153

⇩

170 — Algorithm result provider   ⇐   Ensemble algorithm portion — 155

FIG. 3

# FIG. 4

S110 — Prepare sample

S120 — Align enzyme corresponding to sample and biosensor containing electron transfer mediator

S130 — Apply constant voltage

S140 — Apply cyclic voltage of trigonometric waveform

S150 — Is abnormal signal sensed? — Yes

No

S160 — Calculate blood glucose value using artificial intelligence deep learning-based optimized algorithm

Notify alarm — S170

# FIG. 5

Bias, mg/dL

Existing method

Total,≤15%:98.7% [1026/1040]
100mg/dL ↓, ≤15%:99.7% [359/360]
100mg/dL ↑, ≤15%:98.1% [667/680]

Bias Average : 5.6%
y = 0.018657 × −1.6483

YSI.mg/dL

Bias, mg/dL

Proposed method

Total,≤15%:98.9% [1029/1040]
100mg/dL ↓, ≤15%:99.7% [359/360]
100mg/dL ↑, ≤15%:98.5% [670/680]

Bias Average : 4.9%
y = 0.0081523 × −0.58379

YSI.mg/dL

# FIG. 6

## FIG. 7

FIG. 8

FIG. 9

| 1 | 2 | 3 | 4 | 5 | 6 | 7 | 8 | 9 | 10 | 11 | 12 | 13 | 14 | 15 | 16 | 17 | 18 | 19 | 20 | 21 |

| 1 | 11 | 21 |

# FIG. 10

ADC

Original Signal

Time point (400Hz)

ADC

Downsampled Signal

Time point (400Hz)

FIG. 11

Original Signal

Differential Signal

Integrated Signal

FIG. 12

FIG. 13

```
                        ┌──────────────┐
                        │   Raw data   │
                        └──────────────┘
                               ⇩
                        ┌──────────────┐
              ⇧         │ Preprocessing│         ⇧
                        └──────────────┘
                               ⇩
┌──────────────┐  ┌──────────────┐  ┌──────────────┐
│ Differential │  │ Differential │  │ Differential │
│    signal    │  │    signal    │  │    signal    │
└──────────────┘  └──────────────┘  └──────────────┘
       ⇩                 ⇩                 ⇩
```

| Convolution | Convolution | Convolution | |
| Batch normalization | Batch normalization | Batch normalization | Layer1 |
| ReLU | ReLU | ReLU | |
| Max pooling | Max pooling | Max pooling | |

| Convolution | Convolution | Convolution | |
| Batch normalization | Batch normalization | Batch normalization | Layer2 |
| ReLU | ReLU | ReLU | |
| Max pooling | Max pooling | Max pooling | |

| Convolution | Convolution | Convolution | |
| Batch normalization | Batch normalization | Batch normalization | Layer3 |
| ReLU | ReLU | ReLU | |
| Max pooling | Max pooling | Max pooling | |

| Convolution | Convolution | Convolution | |
| Batch normalization | Batch normalization | Batch normalization | Layer4 |
| ReLU | ReLU | ReLU | |
| Max pooling | Max pooling | Max pooling | |

| Convolution | Convolution | Convolution | |
| Batch normalization | Batch normalization | Batch normalization | Layer5 |
| ReLU | ReLU | ReLU | |
| Max pooling | Max pooling | Max pooling | |

# FIG. 14

# FIG. 15

Full connected layer

Result of feature extraction portion

1x32          1x16          1x8

FIG. 16

# FIG. 17

S210 — | Acquire signal |

S220 — | Pre-process signal |

S230 — | Form algorithm structure formed of feature extraction portion and blood glucose value prediction portion for blood glucose measurement |

S240 — | Learn algorithm |

S250 — | Ensemble algorithm |

<table>
<tr><td colspan="2" align="center"><b>INTERNATIONAL SEARCH REPORT</b></td><td colspan="2">International application No.<br><br><b>PCT/KR2018/004088</b></td></tr>
</table>

| A. | CLASSIFICATION OF SUBJECT MATTER |
|---|---|

*A61B 5/00(2006.01)i, A61B 5/145(2006.01)i, G06N 3/08(2006.01)i*

According to International Patent Classification (IPC) or to both national classification and IPC

| B. | FIELDS SEARCHED |
|---|---|

Minimum documentation searched (classification system followed by classification symbols)

A61B 5/00; A61B 5/145; A61B 5/1495; G01N 15/06; G01N 27/26; G01N 33/49; G01N 33/53; G06F 15/18; G06F 19/00; G06N 3/02; G06N 3/08

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: biometrics, sensor, electrode, deep learning, artificial intelligence

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2017-116505 A1 (MEDTRONIC MINIMED, INC.) 06 July 2017<br>See paragraphs [170]-[174], claim 1 and figure 3. | 1,3,5 |
| Y | | 2,4,6-13,17 |
| A | | 14-16,18 |
| Y | KR 10-0757297 B1 (KMH CO., LTD.) 11 September 2007<br>See paragraph [58]. | 2,7,10,13 |
| Y | KR 10-2016-0032974 A (I-SENS, INC.) 25 March 2016<br>See paragraphs [44]-[60], claims 1, 15 and figures 5-12. | 4,6-10,12 |
| Y | KR 10-2016-0124703 A (KIM, Sung Chun) 28 October 2016<br>See paragraph [130] and claim 1. | 11-13,17 |
| A | KR 10-2013-0050707 A (SAMSUNG ELECTRONICS CO., LTD. et al.) 16 May 2013<br>See the entire document. | 1-18 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br><br>05 NOVEMBER 2018 (05.11.2018) | Date of mailing of the international search report<br><br>**06 NOVEMBER 2018 (06.11.2018)** |
|---|---|
| Name and mailing address of the ISA/KR<br>Korean Intellectual Property Office<br>Government Complex Daejeon Building 4, 189, Cheongsa-ro, Seo-gu,<br>Daejeon, 35208, Republic of Korea<br>Facsimile No. +82-42-481-8578 | Authorized officer<br><br><br>Telephone No. |

Form PCT/ISA/210 (second sheet) (January 2015)

**INTERNATIONAL SEARCH REPORT**
Information on patent family members

International application No.

**PCT/KR2018/004088**

| Patent document cited in search report | Publication date | Patent family member | Publication date |
|---|---|---|---|
| WO 2017-116505 A1 | 06/07/2017 | US 2017-0185733 A1 | 29/06/2017 |
| KR 10-0757297 B1 | 11/09/2007 | None | |
| KR 10-2016-0032974 A | 25/03/2016 | CN 105738430 A | 06/07/2016 |
| | | EP 2998731 A1 | 23/03/2016 |
| | | JP 2016-061772 A | 25/04/2016 |
| | | JP 5933668 B2 | 15/06/2016 |
| | | KR 10-1666978 B1 | 24/10/2016 |
| | | TW 201612515 A | 01/04/2016 |
| | | TW I569009 B | 01/02/2017 |
| | | US 2016-0077037 A1 | 17/03/2016 |
| KR 10-2016-0124703 A | 28/10/2016 | CA 2989538 A1 | 27/10/2016 |
| | | CN 107532211 A | 02/01/2018 |
| | | EP 3287530 A1 | 28/02/2018 |
| | | JP 2018-512871 A | 24/05/2018 |
| | | US 2018-0094308 A1 | 05/04/2018 |
| | | WO 2016-171474 A1 | 27/10/2016 |
| KR 10-2013-0050707 A | 16/05/2013 | US 2013-0117207 A1 | 09/05/2013 |
| | | US 9547820 B2 | 17/01/2017 |

Form PCT/ISA/210 (patent family annex) (January 2015)